# FASCICULE DE BREVET EUROPEEN

(11) **EP 0 820 267 B1**
(45) Date de publication et mention de la délivrance du brevet: **10.04.2002**
(21) Numéro de dépôt: 97901686.2
(22) Date de dépôt: 28.01.1997
(51) Int. Cl.: A61K 7/06

(54) **UTILISATION COSMETIQUE DE MICROFIBRILLES D'ORIGINE NATURELLE ET D'UN POLYMERE FILMOGENE COMME AGENT DE REVETEMENT COMPOSITE DES CHEVEUX, DES CILS, DES SOURCILS OU DES ONGLES**
KOSMETISCHE VERWENDUNG NATÜRLICHER MIKROFIBRILLEN UND EINES FILMBILDENDEN POLYMERS ALS ZUSAMMENGESETZTES UMHÜLLUNGSMITTEL FÜR HAARE, WIMPERN, AUGENBRAUEN UND NÄGEL
COSMETIC USE OF NATURAL MICROFIBRILS AND A FILM-FORMING POLYMER AS A COMPOSITE COATING AGENT FOR HAIR, EYELASHES, EYEBROWS AND NAILS

(30) Priorité: 13.02.1996 FR 9601751
(43) Date de publication de la demande: 28.01.1998
(73) Titulaire: L'OREAL, 75008 Paris (FR)
(72) Inventeur: MONDET, Jean, F-93600 Aulnay-sous-Bois (FR)
(74) Mandataire: Dodin, Catherine
(86) Numéro de dépôt international: FR9700165
(87) Numéro de publication internationale: WO9729734

(56) Documents cités:
- EP-A- 0 379 409
- EP-A- 0 656 176
- WO-A-93/10172
- FR-A- 2 716 887
- PATENT ABSTRACTS OF JAPAN vol. 13, no. 95 (C-573) [3443] , 6 Mars 1989 & JP 63 275507 A (SHISEIDO CO LTD), 14 Novembre 1988,
- INTERN. J. POLYMERIC MATER.,, vol. 11, 1987, pages 229-262, XP000602991 A. BOLDIZAR ET AL: cité dans la demande

## Description

La présente invention concerne l'utilisation d'un polymère filmogène en solution aqueuse ou en dispersion aqueuse et d'une suspension aqueuse de microfibrilles d'origine naturelle sous forme de particules rigides allongées comme agent de revêtement composite des cheveux, des cils, des sourcils ou des ongles dans et pour la préparation d'une composition cosmétique ou dermatologique.

Les compositions cosmétiques ou dermatologiques se présentent, pour la plupart, sous une forme liquide épaissie. Ce type de présentation est très appropriée par le consommateur : il correspond le plus souvent à une préoccupation pratique pour le formulateur : faciliter la prise du produit hors de son conditionnement sans perte significative, limiter la diffusion du produit à la zone locale de traitement, pouvoir répartir le produit de façon régulière sur la zone locale de traitement et pouvoir l'utiliser dans des quantités suffisantes pour obtenir l'effet cosmétique ou dermatologique recherché.

Cet objectif est primordial pour les formulations telles que les compositions capillaires qui doivent s'étaler et se répartir de façon régulière le long des fibres kératiniques et ne pas ruisseler sur le front, la nuque, le visage ou dans les yeux. On recherche particulièrement ce type de présentation pour les produits de coiffage, de maintien et/ou de fixation des cheveux.

Cet objectif est également important pour les compositions aqueuses de maquillage. des cils ou des sourcils qui doivent s'étaler et se répartir de façon régulière le long des cils ou sourcils et ne pas ruisseler sur le visage, dans les yeux ou sur les paupières.

Cet objectif est également important pour vernis à ongles aqueux qui doivent s'étaler et se répartir de façon régulière sur l'ongle sans ruisseler sur le doigt.

On cherche notamment dans l'optique coiffage et/ou maintien des cheveux des gels aqueux capillaires, transparents ou translucides, pouvant se prendre facilement avec les doigts sans effet collant , pouvant s'étaler facilement sur les cheveux et former après séchage un gainage homogène et continu le long du cheveu, sans effet collant au toucher. Les gels aqueux de coiffage et/ou de maintien des cheveux doivent également conduire à un dépôt ayant une adhésion sur le cheveu suffisamment élevée pendant le séchage du gel, au risque d'apporter au cheveu des propriétés mécaniques insuffisantes, une trop faible résistance à la flexion, une mauvaise tenue des cheveux due à un décollage partiel ou total du gainage. Le gainage obtenu par séchage du gel et le maintien de la coiffure en résultant doivent également être résistants à l'humidité ambiante (atmosphère humide, pluie, piscine, ...) et doivent s'éliminer facilement aux shampooings dans la plupart des applications .

La plupart des gels capillaires classiques, par exemple ceux à base de polymères acryliques filmogènes du type CARBOPOL ou PEMULEN ont tendance à former un dépôt hétérogène et discontinu sur le cheveu. Ils sont de plus collants à la prise et sur les cheveux. Leur résistance à l'eau et à l'humidité à l'eau est de plus insuffisante.

D'autres gels capillaires classiques, notamment ceux à base de gomme de guar forment un gainage autour du cheveu qui a tendance à se décoller par endroits pendant le séchage du gel. Leur résistance à l'eau et à l'humidité à l'eau est de plus insuffisante.

La demanderesse a découvert de façon inattendue que l'utilisation d'une suspension aqueuse de microfibrilles d'origine naturelle spécifiques en association avec un polymère filmogène hydroluble en solution ou un polymère filmogène insoluble dans l'eau en dispersion, permettait d'obtenir des formulations capillaires sous forme épaissie et en particulier sous forme de gels aqueux transparents ou translucides, pouvant se prendre facilement avec les doigts sans effet collant, pouvant s'étaler facilement sur les cheveux grâce à un bon effet rhéofluidifiant et pouvant former après séchage un gainage homogène et continu le long du cheveu sans effet collant au toucher. Le gainage ainsi obtenu par cette association est suffisamment adhésif sur le cheveu pendant le séchage ; il est de plus résistant aux agressions mécaniques de l'environnement (frottements, courants d'air) ainsi qu'à l'humidité ambiante. Il s'élimine très facilement aux shampooings.

Dans l'optique des produits aqueux de maquillage des cils et des sourcils (mascaras, ligneurs ou « eye-liners »), on cherche à réaliser des émulsions huile-dans-eau également sous forme épaissie voire sous forme de gel. Ces formulations contiennent en général des pigments en suspension et au moins un polymère filmogène. On recherche des produits visqueux pouvant s'étaler facilement sur les cils ou sourcils sans effet collant aussi bien à l'application que pendant le séchage de la formulation. On recherche aussi des formulations pouvant former après séchage un gainage homogène et continu le long du cil ou des sourcils sans effet collant au toucher. Dans le cadre des mascaras aqueux, le gainage doit permettre un allongement du cil satisfaisant d'un point de vue esthétique. Ledit gainage doit présenter de bonnes propriétés mécaniques notamment une bonne résistance aux frottements à sec et être suffisamment résistant à l'eau, à l'humidité ambiante et aux démaquillants du visage.

La demanderesse a découvert de façon inattendue que l'utilisation d'une suspension aqueuse de microfibrilles d'origine naturelle spécifiques en association avec un polymère filmogène hydrosoluble en solution ou un polymère filmogène insoluble dans l'eau en dispersion, permettait d'obtenir des formulations pour le maquillage des cils ou des sourcils répondant à tous ces objectifs.

Dans le cadre des vernis à ongles aqueux, on cherche à réaliser des suspensions de pigments également sous forme épaissie et notamment de gels aqueux stables, très rhéofluidifiants, facilement étalables sur l'ongle et capables de former rapidement sur l'ongle, après application et séchage, un film ayant une bonne rigidité sans recourir à un agent de coalescence et ne présentant pas d'effet collant au toucher.

La demanderesse a découvert également de façon inattendue que l'utilisation d'une suspension aqueuse de microfibrilles d'origine naturelle spécifiques en association avec un polymère filmogène en dispersion, permettait d'obtenir des formulations pour le maquillage des ongles répondant à tous ces objectifs.

La présente invention a donc pour objet l'utilisation d'un polymère filmogène en solution aqueuse ou en dispersion aqueuse et d'une suspension aqueuse de microfibrilles d'origine naturelle sous forme de particules rigides allongées dont la longueur moyenne est supérieure ou égale à 80 nm et dont le rapport longueur/diamètre (facteur de forme) est de préférence supérieur ou égal à 10, comme agent de revêtement composite des cheveux, des cils, des sourcils ou des ongles dans et pour la préparation d'une composition cosmétique ou dermatologique.

D'autres objets de l'invention apparaîtront à la lumière de la description qui suit.

Les microfibrilles de l'invention se présentent comme des particules allongées élémentaires rigides avec une longueur moyenne de préférence supérieure ou égale à 1 µm et un facteur de forme de préférence supérieur ou égal à 30 et plus particulièrement supérieur ou égal à 60.

Leur taux de cristallinité est généralement supérieur ou égal à 20 % et préférentiellement supérieur à 50%.

Les sources de provenance des suspensions de microfibrilles utilisées selon l'invention sont diverses.

Les microfibrilles d'origine naturelle de l'invention sont de préférence des microfibrilles de cellulose d'origine végétale ou animale ou bien des microfibrilles de chitosane extraite de chitine. Elles peuvent également provenir de transformations bactériennes.

Parmi les microfibrilles cellulosiques d'origine végétale, on peut citer celles extraites de parenchyme, celles extraites du bois, celles extraites de céréales telles que le blé, celles extraites de légumes tels que la betterave ou celles extraites d'algues à parois cellulosiques.

Parmi les microfibrilles cellulosiques d'origine animale, on peut citer, en particulier, celles obtenues à partir de la tunicine de l'enveloppe d'animaux marins appartenant à la famille des tuniciens (par exemple, les espèces comestibles Halocynthia Papillosa du Japon ou Microcosmus Fulcatus de Méditerranée - les violets).

Un certain nombre de publications décrivent ces microfibrilles ainsi que leur mode de préparation.

On peut citer en particulier l'article de V. FAVIER "Polymers for Advanced Technology" vol. 6, 351-55 (1995) et la demande de brevet français n° 2 716 887 relatifs aux microfibrilles de cellulose extraites de tunicine.

On peut citer également l'article de Boldezar et Coll "Prehydrolyzed Cellulose as renforcing filler for thermoplasters" Intern., J. Polymeric Mater., 1987, vol. 11, 229-262, relatif aux microfibrilles de cellulose extraites du bois.

On peut se reférer aussi au document de S. SALMON, AATCC, International Conference, 8-11, 10/95, p 296-306 (1995) pour les microfibrilles de chitosane.

De façon préférentielle, les suspensions aqueuses de microfibrilles d'origine naturelle, conformes à l'invention, sont stabilisées par la présence de charges ioniques qu'elles portent en surface. Ces charges superficielles sont de préférence constituées par des ions sulfates, des ions phosphates ou des ions carboxylates. Elles sont obtenues par un procédé d'extraction qui est décrit dans la demande WO 93/10172. Ce procédé consiste à combiner une désintégration de la cellulose avec un passage dans un homogénéisateur puissant et un traitement acide non hydrolysant par exemple l'acide sulfurique ou l'acide phosphorique dont la fonction est de munir les microfibrilles de charges ioniques en surface sans pour autant modifier le degré initial de polymérisation de la cellulose. Ces charges superficielles peuvent également être dues à un résidu de pectine à la surface des microfibrilles de cellulose.

Les suspensions de microfibrilles utilisées selon l'invention sont généralement translucides voire transparentes à des concentrations inférieures ou égales à 0,3 % en poids. Au-delà de cette valeur, elles deviennent opaques.

Selon l'application cosmétique ou dermatologique envisagée, la concentration en microfibrilles d'origine naturelle varie de préférence de 0,1 à 30 % en poids et plus préférentiellement de 0,2 à 20 % en poids par rapport au poids total de la composition.

Les polymères filmogènes hydrosolubles utilisés conformément à la présente invention peuvent être choisis parmi ceux cités dans les demandes de brevet EP-A-557 196 et EP-A-676 451. Ces polymères sont plus particulièrement utilisés selon l'invention dans les produits capillaires, les mascaras aqueux ou les bases de soin aqueuses pour les ongles.

On peut citer par exemple : l'alcool polyvinylique, l'hydroxyéthylcellulose , les éthers de cellulose généralement utilisés en cosmétique, les gommes de guar, les gommes de caroube.

Suivant le type d'application recherchée, on utilisera préférentiellement
(i) soit un polymère hydrosoluble filmogène synthétique de température de transition vitreuse Tg basse, de préférence inférieure ou égale à 20°C ;
(ii) soit un polymère hydrosoluble filmogène d'origine naturelle (polysaccharides, dérivés de cellulose) de Tg élevée en général supérieure ou égale à 40°C.

Les microfibrilles d'origine naturelle sont présentes dans le mélange microfibrilles/ polymère filmogène hydrosoluble dans des proportions inférieures ou égales à 15 % en poids et plus préférentiellement inférieure ou égale à 10 % en poids.

Les dispersions aqueuses de polymère filmogène insoluble utilisées selon l'invention sont choisies de préférence parmi les latex de polymère hydrophobe ayant une température de transition vitreuse Tg inférieure ou égale à 10°C et plus préférentiellement inférieure ou égale à 5°C.

Les microfibrilles d'origine naturelle de l'invention sont présentes dans le mélange microfibrilles/latex dans des proportions inférieures ou égales à 15 % en poids et plus préférentiellement inférieure ou égale à 10 % en poids.

Ces latex seront plus particulièrement utilisés dans les mascaras aqueux ou les vernis à ongles.

La demanderesse a découvert de façon surprenante plus que l'association suspension de microfibrilles/polymère filmogène dans une composition pour le maquillage des cils contenant des pigments en suspension, améliorait substantiellement l'allongement du cil lorsque la concentration en microfibrilles le mélange microfibrilles/polymère filmogène était supérieure ou égale à 6 % en poids par rapport au polymère filmogène.

Les compositions de l'invention contiennent un milieu cosmétiquement ou dermatologiquement acceptable, c'est-à-dire un milieu compatible avec les ongles, les cheveux, les cils ou les sourcils.

Selon l'application cosmétique ou dermatologique envisagée, la concentration en microfibrilles d'origine naturelle varie de préférence de 0,1 à 30 % en poids et plus préférentiellement de 0,2 à 20 % en poids par rapport au poids total de la composition.

Le milieu cosmétiquement et/ou dermatologiquement acceptable des compositions selon l'invention est plus particulièrement constitué d'eau et éventuellement de solvants organiques cosmétiquement et/ou dermatologiquement acceptables (tolérance, toxicologie et toucher acceptables).

Ces solvants peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des mono-alcools inférieurs linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer des polyols tels que l'isopropylène glycol ; des dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle.

Afin que les compositions cosmétiques ou dermatologiques de l'invention soient plus agréables à utiliser (plus douce à l'application, plus nourrissante, plus émolliente), il est possible d'ajouter une phase grasse dans le milieu de ces compositions.

Cette phase grasse peut comporter une ou plusieurs huiles choisies de préférence dans le groupe constitué par :
- les silicones volatiles ou non-volatiles, linéaires, ramifiées ou cycliques, organomodifiées ou non, hydrosolubles ou non hydrosolubles,
- les huiles minérales telles que l'huile de paraffine et de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah,
- les huiles synthétiques telles que l'huile de Purcellin, les isoparaffines,
- les huiles fluorées et perfluorées,

Elle peut aussi comporter comme matière grasse un(e) ou plusieurs alcools gras, acides gras (acide stéarique) ou cires (paraffine, cires de polyéthylène, camauba, cire d'abeilles).

De façon connue, toutes les compositions de l'invention peuvent contenir des adjuvants habituels dans les domaines cosmétique et dermatologique, d'autres gélifiants et/ou épaississants classiques ; des polymères ; des émulsionnants ; des tensioactifs ; des agents hydratants ; des émollients ; des filtres solaires ; des actifs hydrophiles ou lipophiles ; des agents anti-radicaux libres ; des bactéricides ; des sequestrants ; des antipelliculaires ; des antioxydants ; des conservateurs ; des parfums ; des charges ; des matières colorantes ; des plastifiants ; des agents de coalescence. Les quantités de ces différents adjuvants sont celles classiquement utilisées dans les domaines considérés.

Bien entendu, l'homme de l'art veillera à choisir le ou les éventuels composés à ajouter aux compositions selon l'invention de manière telle que les propriétés avantageuses attachées intrinsèquement aux compositions conformes à l'invention ne soient pas, ou substantiellement pas, altérées par l'addition envisagée.

Les compositions selon l'invention peuvent se présenter sous toutes les formes appropriées pour les cheveux, les cils, les sourcils ou les ongles, notamment sous forme de solutions du type lotion ou sérum ; sous forme de gels aqueux ; sous forme d'émulsions obtenues par dispersion d'une phase grasse dans une phase aqueuse (H/E) ou inversement (E/H), de consistance liquide ou semi-liquide telles que des laits, des crèmes plus ou moins onctueuses, des pâtes. Ces compositions sont préparées selon les méthodes usuelles.

Les compositions selon l'invention peuvent être utilisées comme produits rincés ou comme produits non-rincés capillaires notamment pour le lavage, le soin, le conditionnement, le maintien de la coiffure ou la mise en forme des fibres kératiniques telles que les cheveux.

Elles peuvent être des produits de coiffage tels que des lotions de mise en plis, des lotions pour le brushing, des compositions de fixation et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une mousse pour la fixation ou le traitement des cheveux.

Les compositions de l'invention peuvent être également des shampooings, des compositions à rincer ou non, à appliquer avant ou après un shampooing, une coloration, une décoloration, une permanente ou un défrisage.

Les compositions conformes à l'invention peuvent être des produits pour le maquillage notamment :
- des produits aqueux de revêtement des cils et des sourcils tels que des mascaras "crèmes" ou des mascaras résistants à l'eau (waterproof) ;
- des produits aqueux de revêtement des ongles tels que des bases de soin des ongles, des sous-couches de vernis à solvant classique ou des vernis à ongles aqueux.

Les exemples qui suivent servent à illustrer l'invention sans toutefois présenter un caractère limitatif.

### EXEMPLE 1 : Préparation d'une suspension aqueuse de microfibrilles de cellulose portant en surface des charges sulfates, obtenue à partir de tunicine.

Après avoir nettoyé de manière grossière les morceaux d'enveloppe de violets, ceux-ci sont mis dans 500 ml d'une solution aqueuse de potasse (KOH) à 5 % pendant une nuit. Ils sont ensuite lavés et blanchis pendant 6 heures dans un bain à 80°C, en changeant celui-ci toutes les deux heures. Ce bain est composé de 300 ml d'une solution de chlorite (17 g de NaclO₂ dans 1 I d'eau distillée) mélangée à 300 ml d'une solution tampon acétate (27 g de NaOH ajouté à 75 ml de CH₃COOH et complété jusqu'à 1 I avec de l'eau distillée). Ce traitement de blanchiment est répété 3 fois ; il rend les morceaux de violets complètement blancs.

Les morceaux de cellulose sont ensuite désintégrés dans un mixer Waring Blender, pendant 20 minutes, la concentration en morceaux étant d'environ 5 % dans l'eau distillée. On obtient ainsi une suspension aqueuse floculante de fragments de paroi, que l'on dilue à environ 1 % et qu'on introduit en cycles successifs dans un homogénéisateur mécanique Gaulin 15M8TA. La pression de l'appareil est montée jusqu'à 600 bars, par paliers pour éviter le blocage de l'appareil par les plus gros fragments de cellulose. On surveillera la montée en température qu'on veille à limiter à 70°C. Après une quinzaine de cycles, on obtient une suspension homogène contenant de petits agrégats de fibres. Un simple test d'efficacité de l'opération consiste à observer la montée de l'épaississement du produit. A titre indicatif, le résultat souhaité est atteint lorsqu'une consistance de vaseline est obtenue avec des suspensions titrant environ 2 % de cellulose.

La suspension issue de l'homogénéisateur est alors traitée par l'acide sulfurique, à raison de 300 ml d'acide sulfurique, à raison de 300 ml d'acide sulfurique concentré (95 %) pour 450 ml de la suspension sortant de l'homogénéisateur. On maintient le tout à 60°C pendant 20 minutes. La suspension est ensuite filtrée sur un verre fritté (porosité 1) pour éliminer les gros agrégats, après quoi les fibrilles cellulosiques sont retenues sur un filtre de porosité 4. On lave à l'eau distillée puis à la soude NaOH (0,1 %) jusqu'à la neutralité de la suspension, puis de nouveau à l'eau distillée. La cellulose se dépose alors sur le filtre sous forme d'une pâte aqueuse de consistance visqueuse. On la redisperse dans l'eau, on homogénéise la suspension à l'aide d'un agitateur magnétique, puis on la passe sous ultrasons (Branson Sonifier B12) pendant environ 5 minutes. La suspension finale est prête à l'utilisation, bien homogène, non floculante et stable pendant plusieurs semaines.

### EXEMPLE 2 : Synthèse d'un copolymère hydrosoluble acrylique de transition vitreuse 3°C.

Il s'agit d'un copolymère obtenu par polymérisation radicalaire de :

| | |
|---|---|
| Acide acrylique | 10 % en poids |
| Acrylate d'isobutyle | 70 % en poids |
| Acrylate de t-butyle | 20 % en poids |

La polymérisation est faite en solution dans l'éthanol avec amorçage à l'azo-bis-isobutyronitrile.

Dans un réacteur de 500 ml avec agitation centrale, thermomètre, réfrigérant et tube à barbotage d'azote, on introduit 200 g d'éthanol absolu, 100 g du mélange de monomères et 2 g d'azo-bis-isobutyronitrile. On effectue une agitation à 250 tours/minute et un barbotage à l'azote. On chauffe progressivement jusqu'au reflux (78°C) et on maintient à cette température et dans ces conditions pendant 18 heures. Au bout de ce temps, on ramène à température ambiante et on purifie le polymère par précipitation dans 5 I d'éther de pétrole. On sèche le précipité sous vide à 45° C jusqu'à poids constants. On obtient 81 g de polymère et un rendement de 81 %.

L'indice d'acide mesuré est 81,5.

La viscosité absolue en solution à 5 % en poids de diméthylformamide à 34,6°C est de 1,7 cps.

Le poids moléculaire en sommet de pic de chromatographe par exclusion stérique est de 36.800.

On réalise une solution aqueuse de composition suivante :
- Polymère hydrosoluble filmogène de l'exemple 2 27,37 g
- Amino-2-méthyl-2-propanol (neutralisation à 100 %) 3,53 g
- Eau permutée 69,10 g

### EXEMPLE 3 : Préparation d'un mélange aqueux constitué de l'association de la suspension aqueuse de l'exemple 1 et de la solution de polymère de l'exemple 2.

On ramène la suspension de microfibrilles de l'exemple 1 à un extrait sec de 2,34 % en poids de microfibrilles pour constituer 100 g de gel aqueux.

On prélève 42,65 g de ce gel (suspension de microfibrilles) et on le mélange sous agitation à 57,35 g de solution de polymère de l'exemple 1 pour obtenir un mélange aqueux susceptible de former après séchage un matériau composite comportant 6 % en poids de microfibrilles.

On réalise ainsi un gel aqueux de composition suivante :
- Polymère hydrosoluble filmogène de l'exemple 2 15,7% en poids
- Amino-2-méthyl-2-propanol (neutralisation à 100 %) 2,0% en poids
- Microfibrilles de l'exemple 1 1,0% en poids
- Eau permutée 81,3% en poids

### EXEMPLE 4 : Mascara aqueux

On réalise un mascara par simple addition du gel aqueux de l'exemple 3 avec une quantité suffisante de pigments couramment utilisés pour les mascaras. Le mascara obtenu est stable, homogène, permet d'allonger le cil et présente une bonne rémanence à l'eau. II est facilement éliminable au démaquillant.

### EXEMPLE 5 : Gel capillaire fixant

On réalise un gel capillaire fixant à partir du gel de l'exemple 3. Ce produit donne après évaporation, un film sur les cheveux présentant des propriétés de fixation satisfaisantes, une bonne rémanence à l'eau et facilement éliminable au shampooing.

### EXEMPLE 6 : Synthèse d'un latex filmogène hydrophile

Il s'agit d'un latex de composition :

| | |
|---|---|
| Acide acrylique | 10 % en poids |
| Acrylate d'isobutyle | 70 % en poids |
| Acrylate de t-butyle | 20 % en poids |

Dans un réacteur équipé d'une agitation mécanique centrale, d'un thermomètre et d'un réfrigérant, on introduit 62,5 g d'eau permutée, 1,24 g d'une solution aqueuse à 30,4% de tensio-actif alkyl éthoxy sulfate vendu sous le nom d'ABEX JKB par la Société RHONE POULENC et 0,19 g de persulfate de potassium. On porte le mélange sous agitation rapide à une température de 80°C.

On prépare en parallèle les deux solutions dites « coulées » S₁ et S₂ suivantes :
**coulée S**_{**1**} **(solution de monomères) :**
   - Acide acrylique 12,5 g
   - Acrylate d'isobutyle 87,5 g
   - Acrylate de t-butyle 25 g
   - Dodécane thiol 1,25 g
**coulée S**_{**2**} **:**
   - Eau permutée 312,5 g
   - ABEX JKB solution aqueuse à 30,24% 11,16 g
   - Persulfate de potassium 0,55 g

Lorsque la solution aqueuse dans le réacteur est parvenue à 80°C, on ajoute 10% de la solution S₁ et on laisse réagir 15 minutes. On coule alors pendant une durée de 4 heures, avec un débit constant et simultanément le reste de la solution S₁ et la solution S₂. A la fin des deux ajouts simultanés, on élève la température du milieu réactionnel à 85°C et on maintient à cette température pendant 30 minutes.

On laisse refroidir à température ambiante sous agitation. On filtre sur toile de nylon.

On obtient une dispersion de particules de polymère présentant les caractéristiques suivantes :
- Taille moyenne des particules : 230 nm
- Polydispersité en taille de particules mesurée par diffusion quasi-élastique de la lumière avec un appareil du type COULTER N4 SD : < 0,1.
- Extrait sec en étuve ventilée à 80°C jusqu'à poids constant : 25%
- Poids moléculaire en sommet de pic de chromatographe par exclusion stérique : 70.000.
- Température de transition vitreuse mesurée par DSC : -7°C.

On concentre la dispersion aqueuse de polymère obtenue à l'évaporateur rotatif jusqu'à un extrait sec final de 50,36%.

### EXEMPLE 7 : Préparation d'un mélange aqueux constitué de l'association de la suspension aqueuse de l'exemple 1 et de la dispersion de polymère de l'exemple 6.

On ramène la suspension de microfibrilles de l'exemple 1 à un extrait sec de 2,34 % en poids de microfibrilles pour constituer 100 g de gel aqueux.

On prélève 56,32 g de ce gel (suspension de microfibrilles) et on le mélange sous agitation à 43,68 g de dispersion de polymère de l'exemple 6 pour obtenir un mélange aqueux susceptible de former après séchage un matériau composite comportant 5,7 % en poids de microfibrilles par rapport au polymère.

On réalise ainsi un gel aqueux de composition suivante :
- Polymère filmogène de l'exemple 6 22 g (extrait sec)
- Amino-2-méthyl-2-propanol (neutralisation à 100 %) qs pH7
- Microfibrilles de l'exemple 1 1,32 g
- Eau permutée 76,68 g

### EXEMPLE 8 : Mascara aqueux

On réalise un mascara par simple addition du gel aqueux de l'exemple 7 avec une quantité suffisante de pigments couramment utilisés pour les mascaras. Le mascara obtenu est stable, homogène, permet d'allonger le cil et présente une rémanence à l'eau supérieure à celle du mascara de l'exemple 4. Il est facilement éliminable au démaquillant.

### EXEMPLE 9 : Synthèse d'un latex filmogène hydrophobe

Il s'agit d'un latex de composition :

| | |
|---|---|
| Acrylate d'isobutyle | 63,8 % en poids |
| Acrylate de t-butyle | 36,2 % en poids |

On opère dans les mêmes conditions que celles de l'exemple 6.
On obtient une dispersion de particules de polymère présentant les caractéristiques suivantes :
- Taille moyenne des particules : 250 nm
- Polydispersité en taille de particules mesurée par diffusion quasi-élastique de la lumière avec un appareil du type COULTER N4 SD : < 0,1.
- Extrait sec en étuve ventilée à 80°C jusqu'à poids constant : 24,8%
- Poids moléculaire en sommet de pic de chromatographe par exclusion stérique : 65.000.
- Température de transition vitreuse mesurée par DSC : -3°C.

On concentre la dispersion aqueuse de polymère obtenue à l'évaporateur rotatif jusqu'à un extrait sec final de 49,86%.

### EXEMPLE 10 : Préparation d'un mélange aqueux constitué de l'association de la suspension aqueuse de l'exemple 1 et de la dispersion de polymère de l'exemple 9.

On ramène la suspension de microfibrilles de l'exemple 1 à un extrait sec de 3,5 % en poids de microfibrilles pour constituer 100 g de gel aqueux.

On prélève 42,86 g de ce gel (suspension de microfibrilles) et on le mélange sous agitation cisaillante du type RAINERI à 57,14 g de dispersion de polymère de l'exemple 9 pour obtenir un gel aqueux de composition suivante :
- Polymère filmogène de l'exemple 9 28,5 g (extrait sec)
- Microfibrilles de l'exemple 1 1,5 g
- Eau permutée 70,0 g

La dispersion obtenue contient 5 % en poids de microfibrilles par rapport au poids du polymère.

### EXEMPLE 11 :Vemis à ongles aqueux

On réalise un vemis en dispersant sous agitation cisaillante 1 g de pigments couramment utilisés pour les vernis, dans le gel aqueux de l'exemple 10.

Le vernis obtenu est stable, facile à étaler en raison d'un caractère très rhéofluiodifiant et donnant après séchage un film brillant, de bonne flexibilité sans caractère mou ni effet collant en surface. Le dépôt obtenu sur l'ongle est résistant à la rayure, à l'écaillage présente une bonne adhésion sur l'ongle et une bonne résistance à l'agression de l'eau et aux solutions aqueuses de détergents ou de tensio-actifs.

## Revendications

1. Utilisation d'un polymère filmogène en solution aqueuse ou en dispersion aqueuse et d'une suspension aqueuse de microfibrilles d'origine naturelle sous forme de particules allongées de longueur supérieure ou égale à 80 nm et dont le rapport longueur/diamètre (facteur de forme) est supérieur ou égal à 10, comme agent de revêtement des cheveux, des cils, des sourcils ou des ongles, susceptible de former un dépôt composite après séchage, dans et pour la préparation d'une composition cosmétique ou dermatologique.

2. Utilisation selon la revendication 1, **caractérisée par le fait que** les microfibrilles ont une longueur moyenne supérieure ou égale à 1 µm et un facteur de forme supérieur ou égal à 30 et plus particulièrement supérieur ou égal à 60.

3. Utilisation selon la revendication 1 ou 2, **caractérisée par le fait que** les microfibrilles ont un taux de cristallinité supérieur ou égal à 20 % et de préférence supérieur ou égal à 50 %.

4. Utilisation selon l'une quelconque des revendications 1 à 3, **caractérisée par le fait que** les microfibrilles sont des microfibrilles de cellulose d'origine végétale ou animale, des microfibrilles de chitosane ou des microfibrilles provenant de transformations bactériennes.

5. Utilisation selon l'une quelconque des revendications 1 à 4, **caractérisée par le fait que** les microfibrilles cellulosiques sont choisies parmi celles extraites de parenchyme, de bois, de céréales, de légumes, d'algues à parois cellulosiques ou de tunicine.

6. Utilisation selon l'une quelconque des revendications 1 à 5, **caractérisée par le fait que** les microfibrilles sont porteuses de charges ioniques superficielles.

7. Utilisation selon la revendication 6, **caractérisée par le fait que** les charges ioniques superficielles sont des ions sulfates, des ions carboxylates ou des ions phosphates.

8. Utilisation selon l'une quelconque des revendications 1 à 7, selon laquelle la concentration des microfibrilles dans le mélange microfibrilles/polymère filmogène est inférieure ou égale à 15 % en poids, de préférence inférieure ou égale à 10 % en poids par rapport à la quantité de polymère.

9. Utilisation selon l'une quelconque des revendications 1 à 8, selon laquelle les polymères hydrosolubles filmogènes sont choisis parmi les polymères filmogènes synthétiques de température de transition vitreuse Tg inférieure ou égale à 20°C et les polymères filmogènes d'origine de Tg supérieure ou égale à 40°C.

10. Utilisation selon l'une quelconque des revendications 1 à 8, selon laquelle la dispersion aqueuse de polymère filmogène est un latex de polymère hydrophobe de température de transition vitreuse Tg inférieure à 10° C et de préférence inférieure à 5° C.

11. Utilisation selon l'une quelconque des revendications 1 à 10, dans et pour la préparation d'un produit capillaire pour le lavage, le soin, le conditionnement, le maintien ou la mise en forme des cheveux.

12. Utilisation selon l'une quelconque des revendications 1 à 10, dans et pour la préparation d'un produit de maquillage des cils ou des sourcils.

13. Utilisation selon la revendication 12, où la concentration en microfibrilles dans le mélange microfibrilles/polymère filmogène est supérieure ou égale à 6 % en poids par rapport à la quantité de polymère filmogène.

14. Utilisation selon l'une quelconque des revendications 1 à 10, dans et pour la préparation d'un produit de maquillage et/ou de soin des ongles.

## Claims

1. Use of a film-forming polymer in aqueous solution or in aqueous dispersion and of an aqueous suspension of microfibrils of natural origin in the form of elongated particles whose length is greater than or equal to 80 nm and whose length/diameter ratio (shape factor) is greater than or equal to 10, as coating agent for the hair, the eyelashes, the eyebrows or the nails, which can form a composite deposit after drying, in, and for the preparation of, a cosmetic or dermatological composition.

2. Use according to Claim 1, **characterized in that** the microfibrils have an average length greater than or equal to 1 µm and a shape factor greater than or equal to 30 and more particularly greater than or equal to 60.

3. Use according to Claim 1 or 2, **characterized in that** the microfibrils have a degree of crystallinity greater than or equal to 20 % and preferably greater than or equal to 50 %.

4. Use according to any one of Claims 1 to 3, **characterized in that** the microfibrils are cellulose microfibrils of plant or animal origin, chitosan microfibrils or microfibrils originating from bacterial transformations.

5. Use according to any one of Claims 1 to 4, **characterized in that** the cellulose microfibrils are chosen from those extracted from parenchyma, from wood, from cereals, from vegetables, from algae with cellulosic walls or from tunicin.

6. Use according to any one of Claims 1 to 5, **characterized in that** the microfibrils carry surface ionic charges.

7. Use according to Claim 6, **characterized in that** the surface ionic charges are sulphate ions, carboxylate ions or phosphate ions.

8. Use according to any one of Claims 1 to 7, according to which the microfibril concentration in the microfibrils/film-forming polymer mixture is less than or equal to 15 % by weight, preferably less than or equal to 10 % by weight relative to the amount of polymer.

9. Use according to any one of Claims 1 to 8, according to which the water-soluble film-forming polymers are chosen from synthetic film-forming polymers with a glass transition temperature Tg of less than or equal to 20°C and film-forming polymers of natural origin with a Tg of greater than or equal to 40°C.

10. Use according to any one of Claims 1 to 8, according to which the aqueous dispersion of film-forming polymer is a hydrophobic polymer latex with a glass transition temperature Tg of less than 10°C and preferably less than 5°C.

11. Use according to any one of Claims 1 to 10, in, and for the preparation of, a hair product to wash, care for, condition, maintain or hold the shape of the hair.

12. Use according to any one of Claims 1 to 10, in, and for the preparation of, a make-up product for the eyelashes or the eyebrows.

13. Use according to Claim 12, in which the microfibril concentration in the microfibrils/film-forming polymer mixture is greater than or equal to 6 % by weight relative to the amount of film-forming polymer.

14. Use according to any one of Claims 1 to 10, in, and for the preparation of, a product to make up and/or care for the nails.

## Patentansprüche

1. Verwendung eines filmbildenden Polymers in wäßriger Lösung oder in wäßriger Dispersion und einer wäßrigen Suspension von Mikrofibrillen natürlicher Herkunft, die in Form von länglichen Partikeln mit einer Länge von mindestens 80 nm vorliegen, deren Verhältnis von Länge zu Durchmesser (Formfaktor) mindestens 10 beträgt, als Mittel zum Überziehen von Haaren, Wimpern, Augenbrauen oder Nägeln, das befähigt ist, nach dem Trocknen eine gemischte Abscheidung zu bilden, in einer kosmetischen oder dermatologischen Zusammensetzung oder zur Herstellung einer kosmetischen oder dermatologischen Zusammensetzung.

2. Verwendung nach Anspruch 1, **dadurch gekennzeichnet, daß** die Mikrofibrillen eine mittlere Länge von 1 µm oder darüber und einen Formfaktor von 30 oder darüber und insbesondere mindestens 60 aufweisen.

3. Verwendung nach Anspruch 1 oder 2, **dadurch gekennzeichnet, daß** die Mikrofibrillen einen Kristallinitätsgrad von 20 % oder darüber und vorzugsweise mindestens 50 % aufweisen.

4. Verwendung nach einem der Ansprüche 1 bis 3, **dadurch gekennzeichnet, daß** es sich bei den Mikrofibrillen um Cellulose-Mikrofibrillen pflanzlicher oder tierischer Herkunft, Chitosan-Mikrofibrillen oder Mikrofibrillen handelt, die aus bakteriellen Umwandlungen stammen.

5. Verwendung nach einem der Ansprüche 1 bis 4, **dadurch gekennzeichnet, daß** die Cellulose-Mikrofibrillen unter den Mikrofibrillen ausgewählt sind, die aus dem Parenchym, Holz, Getreide, Gemüse, Algen mit cellulosehaltigen Zellwänden oder Tunicin extrahiert wurden.

6. Verwendung nach einem der Ansprüche 1 bis 5, **dadurch gekennzeichnet, daß** die Mikrofibrillen an der Oberfläche ionische Ladungen tragen.

7. Verwendung nach Anspruch 6, **dadurch gekennzeichnet, daß** die ionischen Ladungen an der Oberfläche Sulfationen, Carboxylationen oder Phosphationen sind.

8. Verwendung nach einem der Ansprüche 1 bis 7, wobei die Konzentration der Mikrofibrillen in dem Gemisch Mikrofibrillen/ film bildendes Polymer 15 Gew.-% oder darunter und vorzugsweise höchstens 10 Gew.-%, bezogen auf die Menge des Polymers, ist.

9. Verwendung nach einem der Ansprüche 1 bis 8, wobei die wasserlöslichen film bildenden Polymere unter den synthetischen filmbildenden Polymeren mit einer Glasübergangstemperatur Tg von 20 °C oder darunter und den film bildenden Polymeren mit einer Tg von 40 °C oder darüber ausgewählt sind.

10. Verwendung nach einem der Ansprüche 1 bis 8, wobei die wäßrige Dispersion des filmbildenden Polymers ein Latex eines hydrophoben Polymers mit einer Glasübergangstemperatur Tg unter 10 °C und vorzugsweise unter 5 °C ist.

11. Verwendung nach einem der Ansprüche 1 bis 10, in einem Produkt für das Haar zum Waschen, zur Pflege, zur Konditionierung, für den Halt oder die Formgebung der Haare oder zur Herstellung eines solchen Produktes.

12. Verwendung nach einem der Ansprüche 1 bis 10, in einem Produkt zum Schminken der Wimpern oder der Augenbrauen oder zur Herstellung eines solchen Produkts.

13. Verwendung nach Anspruch 12, wobei die Konzentration der Mikrofibrillen in dem Gemisch Mikrofibrillen/filmbildendes Polymer mindestens 6 Gew.-%, bezogen auf die Menge des filmbildenden Polymers, ist.

14. Verwendung nach einem der Ansprüche 1 bis 10, in einem Produkt zum Schminken und/oder zur Pflege der Nägel oder zur Herstellung eines solchen Produkts.
